Europäisches Patentamt

**European Patent Office**

**Office européen des brevets**

(19)

(11) Veröffentlichungsnummer: **0 053 244**
A1

## (12) EUROPÄISCHE PATENTANMELDUNG

(21) Anmeldenummer: **81108103.3**

(22) Anmeldetag: **09.10.81**

(51) Int. Cl.³: **A 61 K 31/195**

(30) Priorität: **03.12.80 CH 8925/80**

(43) Veröffentlichungstag der Anmeldung: **09.06.82**
**Patentblatt 82/23**

(84) Benannte Vertragsstaaten: **BE CH DE FR GB IT LI NL SE**

(71) Anmelder: **Dr. Franz Köhler Chemie KG, Neue Bergstrasse 5 - 7, D-6146 Alsbach/Bergstrasse (DE)**

(72) Erfinder: **Köhler, Franz, Dr., Neue Bergstrasse 5, D-6146 Alsbach-Bergstrasse (DE)**

(74) Vertreter: **Zutter, Hans Johann Niklaus, EPROVA AG Forschungsinstitut Im Laternenacker 5, CH-8200 Schaffhausen (CH)**

(54) **Präparat zur Verhütung der EPH-Gestose und andern Stoffwechselstörungen während der Schwangerschaft.**

(57) Die Erfindung betrifft ein Präparat enthaltend Hydrogenasparaginate von Kalium, Magnesium, 3-wertigem Eisen, Zink sowie 2-wertigem Kupfer, Mangan und Cobalt, deren Mengenverhältnis dem physiologischen Bedarf angepasst ist, bzw. dessen Verwendung zur Behandlung der EPH-Gestose und anderen durch Mangel an Enzymaktivatoren verursachten Stoffwechselstörungen während der Schwangerschaft.

EP 0 053 244 A1

- 1 -

Präparat zur Verhütung der EPH-Gestose und andern Stoffwechselstörungen während der Schwangerschaft.

Die Erfindung betrifft ein Kombinationspräparat zur Verhütung der EPH-Gestose und andern durch Mangel an Enzymaktivatoren verursachten Stoffwechselstörungen während der
Schwangerschaft.
EPH-Gestosen sind Schwangerschaftstoxikosen (Gestosen),
die zu Ödemen (Edema), Protunurie und/oder Hypertonie Anlass geben.

Während über den Stellenwert der pharmakodynamischen und
pharmakokinetischen Eigenschaften von Kalium, Natrium,
Calcium und Eisen in der Schwangerschaft eine unübersehbare Literatur vorliegt, werden die funktionellen Eigenschaften der Kationen Magnesium, Zink, Kupfer, Mangan und
Cobalt in Bezug auf den Ablauf der Gravidität erst allmählich erkannt.

Der Serum-Natriumspiegel sinkt während der normalen Gravidität leicht ab, bei pathologischem Verlauf der Schwangerschaft und bei Spätgestosen steigt er an. Der Serum-
Kaliumspiegel liegt bei Schwangeren signifikant höher als
bei Nichtschwangeren.

149/EP

Der **Magnesium**spiegel erfährt in der Gravidität schwere Störungen, die wegen der Funktion als Aktivator bei über 250 Enzymen von besonderer Bedeutung sind. Die Magnesiumkonzentration nimmt im Laufe der Schwangerschaft kontinuierlich ab. Magnesiummangel kann zur Ursache gesteigerter Thrombozytenaggregation (Varizen, Thrombose-Hyperkoagulabilität bei Gestose-Patientinnen) werden und die Synthese der DNS-Doppelhelix beeinträchtigen, sowie vasculärstenokardische, muskuläre, viszerale und zerebrale Krankheitsbilder auslösen. Zur Prophylaxe der EPH-Gestose ist die Normalisierung des Magnesiumhaushaltes erforderlich. Hypomagnesiämie kann Ursache der Asphyxie und der Neugeborenentetanie werden.

Der **Eisen**haushalt in der Gravidität ist in unzähligen Publikationen beschrieben und prophylaktisch im Sinne von mehr oder weniger effizienten Supplementierungsprogrammen berücksichtigt. Auf die Rate der EPH-Gestose hat die Eisenmedikation jedoch keinen signifikanten Einfluss. Mit der Eisenkinetik eng verknüpft ist die pharmakodynamische Wirkung des **Kupfers**, die während der Schwangerschaft signifikanten Entgleisungen unterliegt und deren pathophysiologische Relevanz noch nicht ausreichend erkannt ist. Von hohem Stellenwert ist die Schlüsselrolle dieses Kations als Kupfermetalloprotein der Cytochromoxidase bei der Katalyse des Elektronentransfers von der Atmungskette auf molekularen Sauerstoff.

Der **Zink**haushalt ist für den Bereich der reproduktiven Metabolismen von besonders hoher Bedeutung, wegen der Funktion des Zinks als Metalloenzym der RNS-Polymerase. Zinkmangel limitiert die Prozesse, die mit dem Zellumsatz in Zusammenhang stehen. Hieraus leitet sich das obstretische Risiko ab, das bei Störungen des Zinkstoff-

149/EP

wechsels manifest wird: Zwergwuchs, vermindertes Wachstum, verlangsamte Knochenreifung, gestörte Sexualentwicklung, Hypogonadismus, Eisenmangelanämie, Hepatosplenomegalie, Störungen der Wachstumshormone, verstärkte Hautpigmentierung, verformte Fingernägel und offenbleibende Epiphysenfugen (H.H. Sandsted, R.F. Bork, G.H. Booth, W.J. Darby: Current concepts of Trace Minerals; Med. Clin. od North Am., **54** (1970), 1509; J.A. Halsted, H.A. Ronaghy, P. Abadi, M. Haghshenan, G.H. Amirhakem, R.M. Barakat, J.G. Reinhold: Zinc deficiency in man; the Schiraz experiment; Am. J. Med., **53** (1972), 277). Auch Anomalien des Zentralnervensystems werden mit einem maternalen Zinkdefizit in Zusammenhang gebracht (L.E. Sever, I. Emanuel: Is there a connection between maternal zinc deficiency and congenital malformations of the central nervous system in man? Teratology, 7 (1973), 117.

Mit dem Thema Zink und Schwangerschaft hat sich S. Jameson eingehend beschäftigt. (S. Jameson: Effects of Zinc deficiency in human reproduction: Medical Dissertations Nr. 37, Linköping University, 1976. S. Jameson: Malabsorption, Zinkmangel und Serilität - eine Trais? Praxiskurier, 12.03.1976, 21; Selecta, **20** (1978), 1914. S. Jameson: Diskussionsbeitrag zum Int. Kolloquium über Zinkstoffwechsel - Bedeutung für Klinik und Praxis; herausgegeben von J.D. Kruse-Jarres, TM-Verlag, Bad Oeynhausen, 1979). Der Autor bestätigt die essentielle Funktion des Zinks in der Schwangerschaft.

Der Erfindung lag die Aufgabe zugrunde, ein wirksames Präparat zur Behandlung der zum Teil schweren Entgleisungen des Kationenstoffwechsels mit der Äthiologie und Pathogenese der EPH-Gestose und andern Störungen der Gravidität

- 4 -

0053244

zu finden, die möglicherweise durch Mangel an mineralischen Enzymaktivatoren verursacht werden, wie plazentare
Minderdurchblutungen, Eklampsia convulsiva und Folgeerscheinungen der Belastung des mütterlichen Organismus mit
synthetischen weiblichen Sexualhormonen.
Diese Aufgabe wurde durch Verwendung des nachstehend beschriebenen Kombinationspräparates gelöst. Es wurde gefunden, dass ein Präparat enthaltend Hydrogenasparaginate
von Kalium, Magnesium, 3-wertigem Eisen, Zink sowie 2-wertigem Kupfer, Mangan und Cobalt, deren Mengenverhältnis
dem physiologischen Bedarf angepasst ist, zur Behandlung
der EPH-Gestose und anderen durch Mangel an Enzymaktivatoren verursachten Stoffwechselstörungen während der Schwangerschaft optimal geeignet ist.

Ein solches Präparat ist bereits unter der Bezeichnung
Inzelloval$^{(R)}$ als Geriatrikum bekannt. Es wird bei nachlassender körperlichen und geistigen Leistungsfähigkeit,
verzögerter Rekonvaleszenz und bei Erschöpfungszuständen
angewendet.

Gegenstand der Erfindung ist demnach ein Präparat zur Behandlung der EPH-Gestose und anderen durch Mangel an Enzymaktivatoren verursachten Stoffwechselstörungen während
der Schwangerschaft, dadurch gekennzeichnet, dass es Hydrogenasparaginate von Kalium, Magnesium, 3-wertigem
Eisen, Zink sowie 2-wertigem Kupfer, Mangan und Cobalt
enthält, wobei das Mengenverhältnis dem physiologischen
Bedarf angepasst ist.
Alternativ ist Gegenstand der Erfindung die Verwendung
von Hydrogenasparaginaten von Kalium, Magnesium, 3-wertigem Eisen, Zink sowie 2-wertigem Kupfer, Mangan und Cobalt
in einem dem physiologischen Bedarf angepassten Mengenverhältnis, zur Behandlung der EPH-Gestose und anderen durch
Mangel an Enzymaktivatoren verursachten Stoffwechselstörungen während der Schwangerschaft.

149/EP

Eine Applikationseinheit enthält 50 - 150 mg Kalium- und ebensoviel Magnesium-, 40 - 80 mg Eisen(III)-, 10 - 30 mg Zink-, 2 - 10 mg Kupfer(II)- und 2 - 10 mg Mangan(II) sowie ebensoviel Cobalt(II)-hydrogenasparaginat.

Beispiel 1

Zur klinischen Prüfung wurden 170 Schwangere ab der 8. bis 10. Schwangerschaftswoche bis zur Entbindung mit einem Präparat auf der oben beschriebenen Basis von Chelaten der DL-Asparaginsäure behandelt. Die Dosis betrug 2 x 2 Dragees täglich. In die Kontrollgruppe wurden 176 Schwangere einbezogen, die ab der 8. - 10. Schwangerschaftswoche ein handelsübliches Eisenpräparat erhielten. Bei allen Schwangeren wurde routinemässig kontrolliert: Blutdruck, Hämoglobin, Erythrozytenzahl; in Urin: Sediment, Zucker, Eiweiss, ausserdem: Gewicht, Cardiotokographie, Ultraschall und bei Risikoschwangerschaften die Gesamtöstrogene im 24-Stunden-Sammelurin.

Zusammensetzung des Wirkstoffgemisches:

1 Dragee enthielt

$\quad$ 100 mg Kalium-DL-hydrogensparaginat
$\quad$ 100 mg Magnesium-bis-(DL-hydrogenasparaginat)
$\quad$ 60 mg Eisen(III)-tris-(DL-hydrogenasparaginat)
$\quad$ 10 mg Zink-bis-(DL-hydrogenasparaginat)
$\quad$ 10 mg Kupfer(II)-bis-(DL-hydrogenasparaginat)
$\quad$ 10 mg Cobalt(II)-bis-(DL-hydrogenasparaginat)
$\quad$ 10 mg Mangan(II)-bis-(DL-hydrogenasparaginat)

Zur Auswertung der Ergebnisse hinsichtlich der Häufigkeit des EPH-Syndroms wurden beide Gruppen nach erster, zweiter und dritter Gravidität differenziert und danach die Rate der monosymptomatischen EPH-Gestose (Ödeme allein, Proteinurie allein, Hypertonie allein) sowie der polysymptomatischen EPH-Gestose (Ödeme + Proteinurie, bzw. Ödeme + Hypertonie, bzw. Proteinurie + Hypertonie, bzw. Ödeme + Proteinurie + Hypertonie) aufgeteilt.

149/CH

Tab. 1: Schwangerschaftsverlauf ohne EPH-Gestose

| I. Gravida | | II. Gravida | | III. Gravida | |
|---|---|---|---|---|---|
| Verum | Kontrolle | Verum | Kontrolle | Verum | Kontrolle |
| 63 | 38 | 32 | 25 | 20 | 10 |

Summe: Verum-Gruppe    = 115
       Kontroll-Gruppe =   73

Tab. 2: Schwangerschaftsverlauf mit EPH-Gestose

| | | mono | | | poly | | | |
|---|---|---|---|---|---|---|---|---|
| | | E | P | H | E + P | E + H | P + H | E + P + H |
| I. Gravida | V | 31 | – | – | – | – | – | – |
| | K | 37 | 6 | 2 | 2 | 12 | 2 | 1 |
| II. Gravida | V | 14 | – | – | – | – | – | – |
| | K | 17 | 1 | 5 | 1 | 6 | 1 | 1 |
| III. Gravida | V | 10 | – | – | – | – | – | – |
| | K | 3 | 1 | 1 | 1 | 2 | – | 1 |

V = Verum-Gruppe    =  55
K = Kontroll-Gruppe = 103

Zur übersichtlichen Darstellung des Gesamtergebnisses sind die Zahlenwerte unter Ausserachtlassung der Differenzierung in Erst-, Zweit- und Dritt-Schwangerschaften zusam-

149/CH

mengefasst und in der Tabelle 3 wiedergegeben.

Tab. 3: Gesamtergebnisse des Schwangerschaftsverlaufs

|  | Verum-Gruppe | Kontroll-Gruppe |
|---|---|---|
| n-Schwangerschaften | 170 = 100 % | 176 = 100 % |
| ohne EPH-Gestosen | 115 = 67,6 % | 73 = 41,5 % |
| nur Ödeme | 55 = 32,4 % | 57 = 32,4 % |
| monosympt. EPH-Gestose (Proteinurie oder Hypertension) und polysympt. EPH-Gestose | ∅ | 46 = 26,1 % |
| Eklampsie | ∅ | ∅ |

Die Tabelle 4 stellt die Apgar-Werte der Verum- und Kontrollgruppe dar.

Apgar-Werte werden aufgrund eines Punkte-Schemas zur Beurteilung des Neugeborenen unmittelbar nach der Geburt ermittelt. Es werden Herzschlagfrequenz, Atmung, Muskeltonus, Reflexauslösbarkeit und Hautfarbe 1 Minute nach der Geburt beurteilt und in Punkten 2, 1 oder 0 ausgedrückt. Die Gesamtnotenzahl umfasst die Spanne von 0 bis 10 Punkten [Pschyrembel, klinisches Wörterbuch 185 - 250. Auflage 1968, S. 72].

Tabelle 4 siehe Seite 8.

Sie zeigt, dass die Differenzierung der Apgar-Charakterisierung zugunsten der Verum-Gruppe ausfällt, die bei den Werten von A6, A7, A9 und A10 eine statistisch gesicherte Signifikanz aufweisen.

149/CH

Tab. 4: Apgar-Werte

| Apgar-Wert | Kontrolle | | Verum | |
|---|---|---|---|---|
| | n | % | n | % |
| 10 | 9 | 5.1 | 20 | 11.8 |
| 9 | 78 | 44.3 | 92 | 54.1 |
| 8 | 44 | 25.0 | 38 | 22.4 |
| 7 | 23 | 13.1 | 12 | 7.0 |
| 6 | 9 | 5.1 | 4 | 2.3 |
| 5 | 3 | 1.7 | 2 | 1.2 |
| 4 | 4 | 2.3 | 1 | 0.6 |
| 3 | 3 | 1.7 | - | - |
| 2 | 1 | 0.6 | 1 | 0.6 |
| 1 | 2 | 1.1 | - | - |

Gleich positive Befunde für die Verum-Gruppe ergeben sich aus dem Vergleich der Schwangerschaftsdauer, wie in Tabelle 5 dargestellt.

Tab. 5: Schwangerschaftsdauer

| Kontrolle n | 4 | 3 | - | 4 | 7 | 9 | 10 | 20 | 22 | 21 | 29 | 16 | 14 | 7 | 2 | 4 | 1 | 2 |
|---|---|---|---|---|---|---|---|---|---|---|---|---|---|---|---|---|---|---|
| Verum n | - | 2 | 1 | 2 | - | 4 | 5 | 22 | 21 | 29 | 40 | 22 | 16 | 5 | 2 | - | - | - |
| Abweichung | $-30$ | $-27$ | $-24$ | $-21$ | $-18$ | $-15$ | $-12$ | $-9$ | $-6$ | $-3$ | $\pm 1$ | $+3$ | $+6$ | $+9$ | $+12$ | $+15$ | $+18$ | $+21$ |

149/CH

0053244

Als Nullwert wurde die Graviditätsdauer von 280 Tagen fixiert und Abweichungen davon mit Plus oder Minus eingetragen. Es wird festgestellt, dass die Schwangerschaftsdauer in der Verum-Gruppe eine geringere Streuung um den errechneten Geburtstermin aufweist, als in der Kontroll-Gruppe; d.h., dass unter der Behandlung mit dem erfindungsgemäss beschriebenen Wirkstoffgemisch die Rate der auffallend hohen Anzahl Frühschwangerschaften erheblich geringer ist, als in dem Kontrollkollektiv.

Wir haben in einer vergleichenden Untersuchung von 346 Graviden (170 Verum versus 176 Kontrollen) gefunden, dass sich die Häufigkeitsrate der monosymptomatischen EPH-Gestosen ohne Ödeme (d.h. entweder nur Proteinurie oder nur Hypertonie) und der polysymptomatischen EPH-Gestosen durch konsequente Substitution mit essentiellen Kationen in Form ihrer asparaginsauren Chelate auf Null reduzieren lässt, während in der Kontrollgruppe die Rate der EPH-Gestosen bei 26.1 % liegt, in Uebereinstimmung mit dem Ergebnis einer retrospektiven Studie von H. Riedel, H. Weitzel, P. Ritz über "Indikation zur Sectio bei schwerer EPH-Gestose"; Ref. bei der 86. Tagung der Nordwestdeutschen Ges. f. Gynäkologie, Kiel, Mai 1979, die für die Jahre 1969-1977 bei 12.751 Geburten eine EPH-Gestose-Häufigkeit von 22.6 % aufweist. Frei von EPH-Gestosen bleiben in der Verum-Gruppe 67.6 % der Schwangeren, in der Kontroll-Gruppe 41.5 %. In beiden Gruppen liegt die Ödem-Rate bei 32.4 %.

Diese sehr überzeugenden Resultate liegen weit über der Erwartung.

149/CH

0053244

Patentansprüche:

1. Präparat zur Behandlung der EPH-Gestose und anderen durch Mangel an Enzymaktivatoren verursachten Stoffwechselstörungen während der Schwangerschaft, **dadurch gekennzeichnet**, dass es Hydrogenasparaginate von Kalium, Magnesium, 3-wertigem Eisen, Zink sowie 2-wertigem Kupfer, Mangan und Cobalt enthält, wobei das Mengenverhältnis der Kationen dem physiologischen Bedarf angepasst ist.

2. Verwendung von Hydrogenasparaginaten von Kalium, Magnesium, 3-wertigem Eisen, Zink, sowie 2-wertigem Kupfer, Mangan und Cobalt in einem dem physiologischen Bedarf angepassten Mengenverhältnis, zur Behandlung der EPH-Gestose und anderen durch Mangel an Enzymaktivatoren verursachten Stoffwechselstörungen während der Schwangerschaft.

3. Präparat nach Patentanspruch 1 und dessen Verwendung nach Patentanspruch 2, **dadurch gekennzeichnet**, dass eine Applikationseinheit 509- 150 mg Kalium- und ebensoviel Magnesium-, 40 - 80 mg Eisen(III)-, 10 - 30 mg Zink-, 2 - 10 mg Kupfer(II)- und 2 - 10 mg Mangan(II)-, sowie ebensoviel Cobalt(II)-hydrogenasparaginat enthält.

149/EP

**EUROPÄISCHER TEILRECHERCHENBERICHT,** der nach Regel 45 des Europäischen Patent-übereinkommens für das weitere Verfahren als europäischer Recherchenbericht gilt

Europäisches Patentamt

0053244

Nummer der Anmeldung

EP 81 10 8103

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| DX | ROTE LISTE, 1971, Ausg. Cantor Aulendorf/Württ., DE. <br> * Seite 640, "Inzelloval" * <br> -- | 1,3 |
| X | ROTE LISTE, 1971, Ausg. Cantor Aulendorf/Württ., DE. <br> * Seite 640, "Inzolen" * <br> -- | 1,3 |
| X | ROTE LISTE, 1965, Ausg. Cantor Aulendorf/Württ., DE. <br> * Seite 579, "Intra-Ion" * <br> -- | 1,3 |
| A | FR - M - 3209M (EDOUARD PAUL GRENET) <br> * Seiten 1,2, Zusammenfassung * <br> ---- | 1,3 |

**KLASSIFIKATION DER ANMELDUNG (Int. Cl.³)**

A 61 K 31/195

**RECHERCHIERTE SACHGEBIETE (Int. Cl.³)**

A 61 K

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1,3

Unvollständig recherchierte Patentansprüche:

Nicht recherchierte Patentansprüche: 2: Verfahren zur chirur-

Grund für die Beschränkung der Recherche: gischen oder thera-peutischen Behandlung des menschlichen oder tierischen Körpers. (Siehe Art. 52(4) des Europäischen Patentübereinkommens).

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde lie-gende Theorien oder Grund-sätze
E: älteres Patentdokument, das jedoch erst am oder nach dem Anmeldedatum veröffentlicht worden ist
D: in der Anmeldung angeführtes Dokument
L: aus andern Gründen ange-führtes Dokument

&: Mitglied der gleichen Patent-familie, übereinstimmendes Dokument

| Recherchenort | ßdatum der Recherche | Prüfer |
|---|---|---|
| Den Haag | 18-02-1982 | BRINKMANN |

EPA Form 1505.1   06.78